# EUROPEAN PATENT APPLICATION

(11) **EP 4 846 999 A2**
(43) Date of publication of application: **02.12.2026**
(21) Application number: 26210591.9
(22) Date of filing: 06.07.2022
(51) Int. Cl.: A61J 1/14

(54) **SMALL-VOLUME CRYOGENIC STORAGE CONTAINER**

(30) Priority: 06.07.2021 US 202163218550 P
(62) Document number(s) of the earlier application(s) in accordance with Art. 76 EPC: 22838349.3 / 4 366 526
(71) Applicant: BioLife Solutions, Inc., Bothell, WA 98021 (US)
(72) Inventor: The designation of the inventor has not yet been filed
(74) Representative: Götz, Gudrun Veronika

(57) **Abstract**

A cryovial device is disclosed including a vial configured to hold a liquid sample and an inlet/outlet tube coupled to the vial. The inlet/outlet tube is constructed of a weldable polymer and has a filled configuration, a closed configuration, and a drained configuration.

## Description

### REFERENCE TO RELATED APPLICATIONS

This application claims an invention disclosed in U.S. Provisional Application Number 63/218,550, filed July 6, 2021, entitled "Small-Volume Cryogenic Storage Container". Benefit under 35 USC §119(e) of the United States provisional application is claimed, and the aforementioned application is incorporated herein by reference.

### FIELD OF THE DISCLOSURE

The present disclosure relates to cryopreservation. More particularly, the present disclosure relates to a cryovial device and to a method for using the same.

### BACKGROUND OF THE DISCLOSURE

Cryopreservation is the process of cooling and storing biological material (e.g., cells, tissues, organs) at very low temperatures to maintain their viability for future use. The biological material's post-thaw function should be sufficiently representative of the biological material's pre-freeze function.

Cryovials are commonly used for cryopreservation. Such cryovials should be capable of withstanding cryogenic temperatures while also avoiding contamination or leakage of the biological material. Such cryovials should also be efficient and compatible for use in different laboratory and clinical settings.

### SUMMARY

A cryovial device is disclosed including a vial configured to hold a liquid sample and an inlet/outlet tube coupled to the vial. The inlet/outlet tube is constructed of a weldable polymer and has a filled configuration, a closed configuration, and a drained configuration.

According to an exemplary embodiment of the present disclosure, a cryovial device is disclosed including a vial configured to hold a liquid sample, an inlet/outlet tube coupled to the vial and constructed of a weldable polymer, the inlet/outlet tube having a filled configuration in which the inlet/outlet tube is coupled to a source of the liquid sample, and a drained configuration in which the inlet/outlet tube is coupled to a receiving tube, and a vent tube coupled to the vial.

According to another exemplary embodiment of the present disclosure, a method of using a cryovial device is disclosed including a vial and an inlet/outlet tube. The method includes the steps of filling the vial with a liquid sample via the inlet/outlet tube, closing the inlet/outlet tube after the filling step, cryopreserving the sample in the vial after the closing step, opening the inlet/outlet tube after the cryopreserving step, coupling the inlet/outlet tube to a receiving tube, and draining the sample from the vial into the receiving tube via the inlet/outlet tube.

### BRIEF DESCRIPTION OF THE DRAWINGS

The above-mentioned and other features and advantages of this disclosure, and the manner of attaining them, will become more apparent and will be better understood by reference to the following description of embodiments of the invention taken in conjunction with the accompanying drawings, wherein:
FIG. 1 is a perspective view of an exemplary cryovial device of the present disclosure;
FIG. 2 is a front elevational view of the cryovial device of FIG. 1;
FIG. 3 is a side elevational view of the cryovial device of FIG. 1;
FIG. 4 is a perspective view of an optionally used seal element unassembled with the cryovial device of FIG. 1.
FIG. 5 is a perspective view of the seal element of FIG. 4 assembled to the cryovial device of FIG. 1, with a portion of the cryovial device of FIG. 1 removed to view the seal element of FIG. 4.
FIG. 6 is a perspective view of the cryovial device of FIG. 1 additionally including the seal element of FIG. 4.
FIG. 7 is an elevational view of a storage container for holding one or more of the cryovial devices of FIG. 1; and
FIG. 8 shows a method of using the cryovial device of FIG. 1, the method including a filling step (a), a closing step (b), a severing step (c), an unraveling step (d), an opening step (e), a coupling step (f), and a draining step (g).

Corresponding reference characters indicate corresponding parts throughout the several views. The exemplifications set out herein illustrate exemplary embodiments of the invention and such exemplifications are not to be construed as limiting the scope of the invention in any manner.

### DETAILED DESCRIPTION

### Cryovial Device

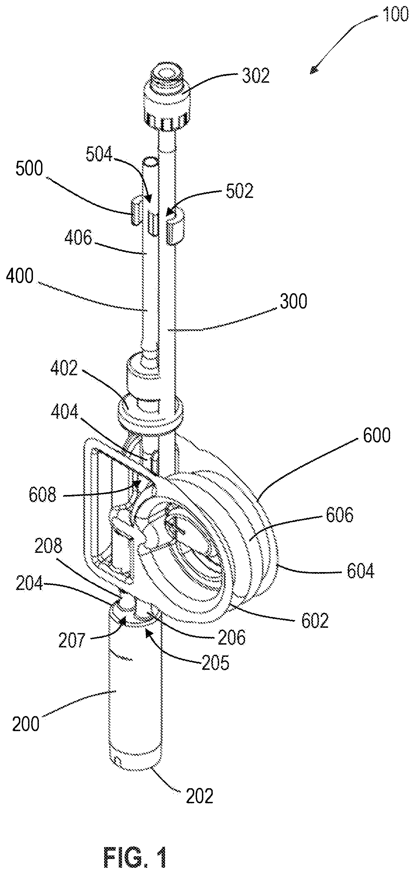

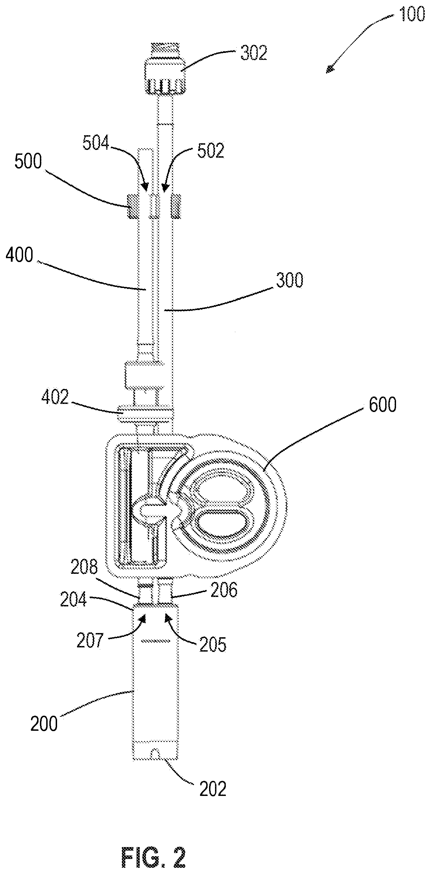

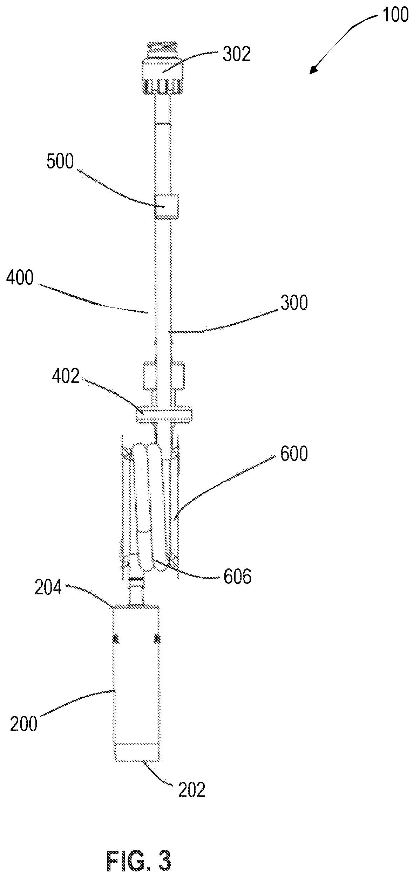

A cryovial device 100 is shown in FIGS. 1-3. The cryovial device 100 is configured to receive a liquid sample, contain the sample during cryostorage, and deliver the thawed sample. The sample may include a biological fluid, such as a suspension of blood cells (e.g., hematopoietic stem and progenitor cells (HPCs) derived from premature cord blood (PCB)). The sample may also include electrolytes and/or cryoprotectants (e.g., glycerol, propylene glycol, ethylene glycol, dimethyl sulfoxide (DMSO)). The cryovial device 100 may be considered a substantially closed system with fluid-tight materials and joints that are capable of withstanding cryogenic temperatures (e.g., about -196° C).

The illustrative cryovial device 100 of FIGS. 1-3 includes a vial 200, a first, inlet/outlet tube 300, a second, vent tube 400, a tube clip 500, and a spool 600. Each element of the cryovial device 100 is described further below.

The vial 200 of the illustrative cryovial device 100 is configured to contain the sample. The illustrative vial 200 is configured to hold about 2mL to about 5 mL of the sample, although this volume may vary from about 1 mL to about 30 mL or more. The illustrative vial 200 is cylindrical in shape, although this shape may also vary. The vial 200 has a closed lower end 202 and an upper end 204 with a first, inlet/outlet opening 205 and a second, vent opening 207. The first, inlet/outlet opening 205 is defined by a first fitting 206, which is configured to couple to the first, inlet/outlet tube 300. The second, vent opening 207 is defined by a second fitting 208, which is configured to couple to the second, vent tube 400. The illustrative fittings 206, 208 are barbed and configured to be friction-fit within their respective tubes 300, 400, but it is also within the scope of the present disclosure for the fittings 206, 208 to be heat-sealed, molded, adhered, and/or otherwise coupled to their respective tubes 300, 400. The first fitting 206 is illustratively taller than the second fitting 208, although this arrangement may vary. The vial 200 may be constructed of a rigid material such as polystyrene, polypropylene, or another suitable material.

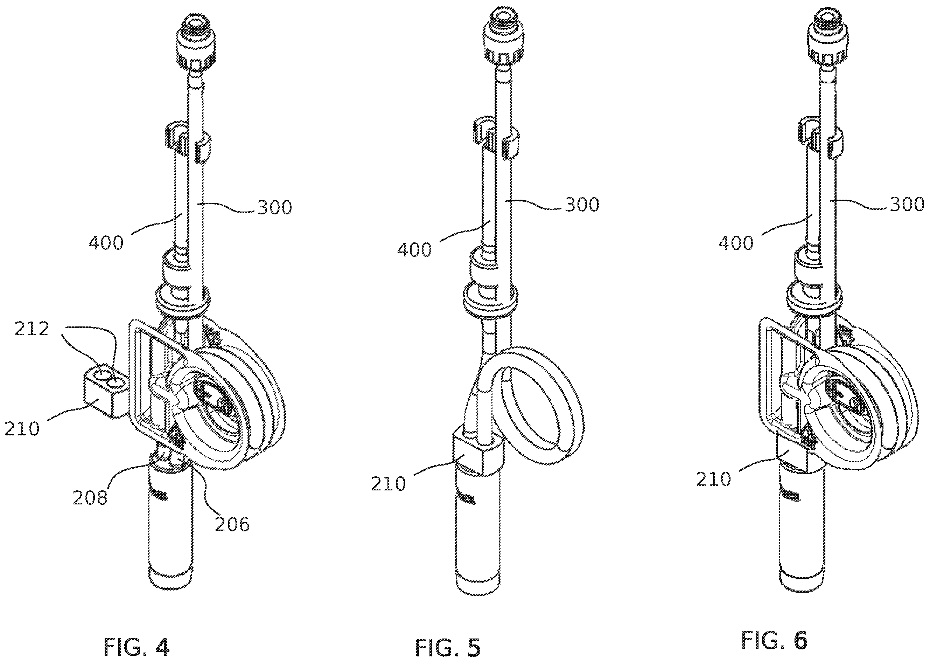

In some embodiments, a sealing element 210 can be used, as illustrated in FIGS. 4-6, to facilitate securing the inlet/outlet tube 300 and the vent tube 400 to the respective fittings 206, 208, and to reduce or prevent fluid leakage between the tubes 300, 400 and their respective fittings 206, 208. The sealing element 210 can have two holes 212 spaced in correspondence with the spacing between the inlet/outlet tube 300 and the vent tube 400. The holes 212 can be of a size or diameter for a friction or interference fit around the inlet/outlet tube 300 and the vent tube 400, to squeeze or compress the tubes 300, 400 around their respective fittings 206, 208. The friction or interference fit can be accomplished by fabricating the sealing element 210 from an elastomeric material that stretches and compresses around the tubes 300, 400, a heat-shrink material that shrinks to compress around the tubes 300, 400, or a non-elastomeric material such as plastic or metal.

Referring again to FIGS. 1-3, the inlet/outlet tube 300 of the illustrative cryovial device 100 is configured to both receive the liquid sample and deliver the thawed sample through the inlet/outlet opening 205 of the vial 200. In this way, the dual-purpose inlet/outlet tube 300 and its corresponding, dual-purpose inlet/outlet opening 205 may eliminate the need for distinct inlet and outlet openings in the vial 200. Initially, the inlet/outlet tube 300 may be provided with a desired fill port 302. The illustrative fill port 302 is a needle-free, female Luer fitting having a normally closed diaphragm valve that opens when coupled to an industry-standard, male Luer fitting. However, the fill port 302 may vary based on the intended application. For example, the fill port 302 may include a needle septum configured to be pierced by a syringe needle. The first, inlet/outlet tube 300 may be longer than the second, vent tube 400, and this excess length may be wrapped around the spool 600, as discussed further below. The inlet/outlet tube 300 may be constructed of a flexible, pharmaceutical grade, weldable polymer. For example, the inlet/outlet tube 300 may be constructed of a thermoplastic elastomer (TPE) tubing, such as Tygon^{®} tubing available from Saint-Gobain Performance Plastics.

The vent tube 400 of the illustrative cryovial device 100 is configured to vent gas into and/or from the vial 200 through the second, vent opening 207 while remaining liquid-tight. For example, the vent tube 400 may allow air to pass from the vial 200 during filling and into the vial 200 during draining. The vent tube 400 includes a filter element 402 along its length that is configured to filter the air entering the vial 200 during draining and/or at other times. The illustrative filter element 402 is positioned about midway along the length of the vent tube 400 between a lower tube portion 404 and an upper tube portion 406, although the location of the filter element 402 may vary. The filter element 402 may be a micro-filter, such as a 3 µm sterile micro-filter. The filter element 402 may be gas permeable but liquid impermeable to avoid leakage of the sample from the vial 200. The vent tube 400, like the inlet/outlet tube 300, may be constructed of a flexible, pharmaceutical grade, thermoplastic elastomer (TPE) tubing, such as Tygon^{®} tubing available from Saint-Gobain Performance Plastics.

The tube clip 500 of the illustrative cryovial device 100 is configured to support and stabilize the tubes 300, 400. The tube clip 500 may be a "3"-shaped component including a first recess 502 configured to hold the first, inlet/outlet tube 300, and a second recess 504 adjacent to the first recess 502 and configured to hold the second, vent tube 400. The tube clip 500 may be sized to slide along the tubes 300, 400 and may be detached from the tubes 300, 400, such as by pinching and removing the tubes 300, 400.

The spool 600 of the illustrative cryovial device 100 is configured to support and stabilize the first, inlet/outlet tube 300. The spool 600 may be constructed of a first portion 602 and a second portion 604 that are snap-fit together. The spool 600 may include a barrel 606 configured to receive the first, inlet/outlet tube 300 in a coiled manner. The spool 600 may also include a passageway 608 configured to freely receive the second, vent tube 400.

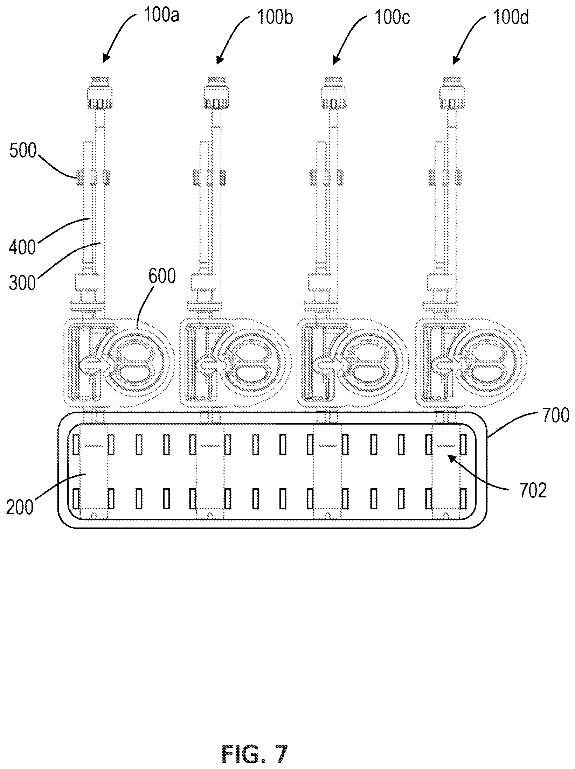

Referring next to FIG. 7, the illustrative cryovial device 100 may be sized for receipt in a standard, tray-shaped, "egg carton" type storage container 700 used to transfer and store cell samples for freezing and eventual thawing. For example, the vial 200 of the cryovial device 100 may be sized for receipt in a separated area 702 of the storage container 700 having a diameter of about 10 mm and a height of about 90 mm. The tubes 300, 400 may project upward from the vial 200 and the storage container 700, supported by the tube clip 500 and/or the spool 600. As shown in FIG. 7, several such cryovial devices 100, illustratively cryovial devices 100a-100d, carrying cell samples from a common source may be arranged in an array and housed in a common storage container 700.

### Method of Use

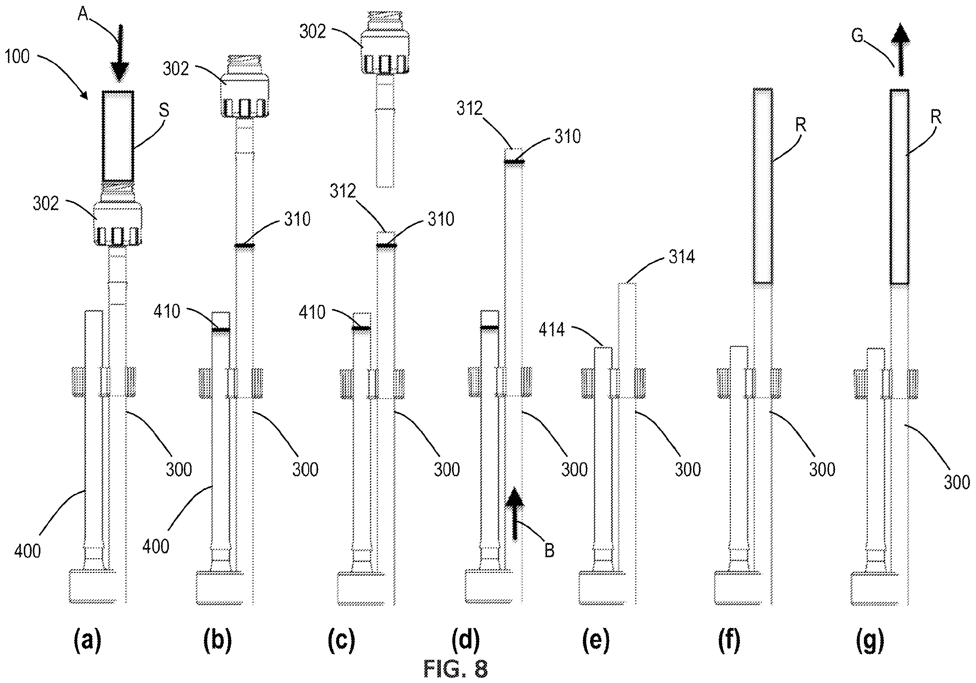

An exemplary method of using the cryovial device 100 is demonstrated in FIG. 8 and described below. During some or all of the following steps, the vial 200 may be present in the above-described storage container 700 (FIG. 7) with the tubes 300, 400 supported by the tube clip 500 and/or the spool 600.

The method of FIG. 8 begins with a filling step (a) with the cryovial device 100 in a filled configuration. During this filling step (a), the sample is transferred from a source S, through the inlet/outlet tube 300, and into the inlet/outlet opening 205 of the vial 200 (FIG. 1), as indicated by arrow A. The source S may be a syringe, a blood bag, or another suitable container for the sample. In certain embodiments, the source S may be present in an automated filling system, such as the CellSeal^{®} AF-500^{™} filling system or the Signata CT-5^{™} filling system, both available from Sexton Biotechnologies. The source S may be coupled (e.g., Luer-locked) to the inlet/outlet tube 300 via the fill port 302, as shown in FIG. 8. Alternatively, the fill port 302 may be removed, and the source S may be coupled (e.g., welded) to the inlet/outlet tube 300 in a direct, closed manner. The sample may be introduced under the influence of gravity, positive pressure from the source S, and/or vacuum pressure through the vent tube 400. Air may escape from the vial 200 via the vent tube 400 during this filling step (a).

The method of FIG. 8 continues with a closing step (b) with the cryovial device 100 in a closed configuration. During this closing step (b), the inlet/outlet tube 300 is heat-sealed or otherwise closed at seal 310 and the vent tube 400 is heat-sealed or otherwise closed at seal 410 to contain the sample in the cryovial device 100. The seal 310 may be located between the first fitting 206 of the vial 200 (FIG. 1) and the fill port 302 of the inlet/outlet tube 300 and above the height of the vent tube 400 to avoid interfering with the vent tube 400. The seal 410 may be located above the filter element 402 (FIG. 1) of the vent tube 400. The closing step (b) may be performed using a medical-grade tube sealer that pinches and welds the inlet/outlet tube 300, such as the C'EAL-FLEX^{®} TPE Ultra Sealer available from Saint-Gobain.

The method of FIG. 8 continues with a severing step (c) with the cryovial device 100 in a severed configuration. During this severing step (c), the excess inlet/outlet tube 300 is sliced along cut line 312 at or above the seal 310 and removed. This severing step (c) may be performed substantially simultaneously with the above-described closing step (b) in a closed environment. For example, both the closing step (b) and the severing step (c) may be performed using the above-described tube sealer.

The method of FIG. 8 continues with an unraveling step (d) with the cryovial device 100 in an unraveled configuration. During this unraveling step (d), the inlet/outlet tube 300 is unraveled from the spool 600 (FIG. 1), as indicated by arrow B. This unraveling step (d) gives the inlet/outlet tube 300 added length and clearance above the vent tube 400.

With the inlet/outlet tube 300 sealed, the sample in the cryovial device 100 may be processed. For example, the sample may be cryogenically frozen, stored/banked, and eventually thawed. It is also within the scope of the present disclosure for the sample to be transported, tested (e.g., cell count analysis, hemoglobin analysis, infectious disease screening, human leukocyte antigen (HLA) typing), and/or otherwise processed. During these processing steps, and as described above with respect to FIG. 7, the vial 200 may be supported by the above-described storage container 700, and the tubes 300, 400 may be supported by the tube clip 500 and/or the spool 600.

The method of FIG. 8 continues with an opening step (e) with the cryovial device 100 in an opened configuration and a coupling step (f) with the cryovial device 100 in a coupled configuration. During the opening step (e), the inlet/outlet tube 300 is sliced along cut line 314 below the seal 310, and the vent tube 400 is sliced along cut line 414 below the seal 410 but still above the filter element 402 (FIG. 1). In this way, the inlet/outlet tube 300 becomes progressively shorter from the filling step (a), to the severing step (c), to the opening step (e). During the coupling step (f), the now-opened end of the inlet/outlet tube 300 is coupled (e.g., welded) to a receiving tube R in a direct, closed manner. The opening step (e) and the coupling step (f) may be performed substantially simultaneously in a closed environment to avoid leakage and/or contamination of the sample. For example, both the opening step (e) and the coupling step (f) may be performed using a tubing welder that cuts and heats adjoining ends of the inlet/outlet tube 300 and the receiving tube R, such as the CONNECT-FLEX^{®} TPE Tubing Welder available from Saint-Gobain. The opening step (e) and the coupling step (f) of the inlet/outlet tube 300 may be performed above the height of the vent tube 400 to avoid interfering with the vent tube 400. If necessary, the inlet/outlet tube 300 may be unraveled further from the spool 600 (FIG. 1) for added length and clearance above the vent tube 400.

The method of FIG. 8 concludes with a draining step (g) with the cryovial device 100 in a drained configuration. During the draining step (g), the sample is directed from the inlet/outlet opening 205 of the vial 200 (FIG. 1), through the inlet/outlet tube 300, and through the receiving tube R, as indicated by arrow G. The draining step (g) may be performed at atmospheric pressure, with air entering the vial 200 via the reopened vent tube 400 and its corresponding filter element 402 (FIG. 1). The withdrawn sample may be directed to its desired end use, such as laboratory testing or clinical administration. In this way, the sample travels through the same inlet/outlet tube 300 in opposite directions during the draining step (g) and the above-described filling step (a). The drained cryovial device 100 may be discarded.

While this invention has been described as having exemplary designs, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. A cryovial device comprising:
a vial configured to hold a liquid sample;
an inlet/outlet tube coupled to the vial and constructed of a weldable polymer, the inlet/outlet tube configured to be coupled to a source of the liquid sample to fill the vial and configured to be coupled to a receiving tube to drain the vial; and
a vent tube coupled to the vial.

2. The cryovial device of claim 1, wherein the inlet/outlet tube is coupled to the vial at a first fitting and the vent tube is coupled to the vial at a second fitting.

3. The cryovial device of claim 2, further comprising a sealing element, the sealing element including a first hole, the first hole receiving the inlet/outlet tube and compressing the inlet/outlet tube around the first fitting, the sealing element including a second hole, the second hole receiving the vent tube and compressing the vent tube around the second fitting.

4. The cryovial device of claim 1, wherein the inlet/outlet tube is constructed of a thermoplastic elastomer.

5. The cryovial device of claim 1, wherein the inlet/outlet tube is welded to the receiving tube in the drained configuration.

6. The cryovial device of claim 1, wherein the inlet/outlet tube is configured to be closed.

7. The cryovial device of claim 6, wherein the inlet/outlet tube is configured to be heat-sealed to close the inlet/outlet tube.

8. The cryovial device of claim 6, wherein the inlet/outlet tube is configured to be heat-sealed to close the inlet/outlet tube beyond an extension of the vent tube from the vial.

9. The cryovial device of claim 6, wherein the vent tube is configured to be heat-sealed to close the vent tube.

10. The cryovial device of claim 1, wherein the inlet/outlet tube is-longer than the vent tube.

11. The cryovial device of claim 1, further comprising a spool around which the inlet/outlet tube is wrapped.

12. A method of using a cryovial device including a vial and an inlet/outlet tube, the method comprising the steps of:
filling the vial with a liquid sample via the inlet/outlet tube;
closing the inlet/outlet tube after the filling step;
cryopreserving the sample in the vial after the closing step;
opening the inlet/outlet tube after the cryopreserving step;
coupling the inlet/outlet tube to a receiving tube; and
draining the sample from the vial into the receiving tube via the inlet/outlet tube.

13. The method of claim 12, further comprising the step of unraveling the inlet/outlet tube from a spool after the closing step.

14. The method of claim 12, wherein the closing step comprises heat-sealing the inlet/outlet tube.

15. The method of claim 12, further comprising the step of severing the inlet/outlet tube.

16. The method of claim 12, wherein the coupling step comprises welding the inlet/outlet tube to the receiving tube.
